# EUROPEAN PATENT SPECIFICATION

(11) **EP 1 514 511 B1**
(45) Date of publication and mention of the grant of the patent: **21.05.2008**
(21) Application number: 03020615.5
(22) Date of filing: 10.09.2003
(51) Int. Cl.: A61B 5/0235

(54) **Air pressure adjusting device**
Vorrichtung zur Regelung des Luftdrucks
Dispositif pour le réglage de la pression de l'air

(43) Date of publication of application: 16.03.2005
(73) Proprietor: Rossmax International LTD., Taipei (TW)
(72) Inventor: Chen, Chin-Jung, Taipei Taiwan 114, R.O.C. (TW)
(74) Representative: Schaeberle, Steffen

(56) References cited:
- EP-A- 1 298 371
- JP-A- 6 047 011
- SU-A- 783 051
- US-B1- 6 346 082

## Description

### BACKGROUND OF THE INVENTION

### Field of the Invention

The invention relates in general to an air pressure adjusting device according to claim 1, and more particularly to an air pressure adjusting device, which uses a force on the outlet exerted as a result of the deformation of a deformation element to adjust the air pressure in a container.

### Description of the Related Art

Studies have shown that, of all the hospital diagnostic tests, blood pressure tests are made the most frequently. However, blood-pressure measurements may be a little inaccurate. Typically, blood pressure is measured by using an aneroid monitor. By placing the stethoscope disk at the brachial artery, wrapping the bladder of the cuff snugly around the upper arm, and then squeezing the bulb to pump air into the cuff bladder, the mercury level of a blood pressure gauge will rise slowly. Pumping is stopped at a pressure of about 180 mmHg, temporarily stopping blood flow to the brachial artery. Air in the bladder is then released slowly at a stable rate (i.e. the bladder is deflated). Meanwhile, the mercury level in the pressure gauge is observed and heart sounds carefully monitored. When something that sounds like "prrpshh" (the sound of the heartbeat) is heard or appears for the first time, that is blood starts flowing in the artery again, the air pressure in the bladder is considered as the systolic blood pressure. The sounds continue and become louder in intensity. When the sound is heard for the last time or disappears, indicating a return to normal blood flow, the air pressure in the bladder is considered as the diastolic blood pressure. After the readings are taken, air in the bladder is released completely. Clearly, if air is released from the bladder too rapidly, blood pressure may not be determined. Therefore, an apparatus of controlling the release rate of air in the bladder will improve the precision of the readings from the pressure gauge.

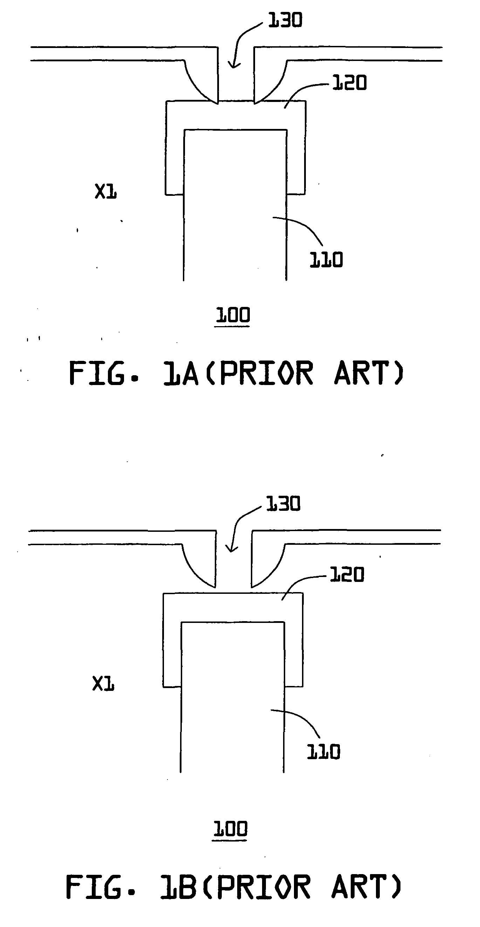

Referring to FIG. 1A, a schematic view of a conventional air pressure-adjusting device with the shifting element adjoined to the bladder outlet is shown. The conventional air pressure-adjusting device 100 includes a driving element (not shown in FIG. 1A), a shifting element 110, and a deformation element 120.

The shifting element 110, coupled to the driving element (such as an electromagnetic coil), is driven to move along the shifting direction X1 by the driving element. The deformation element 120, disposed on the shifting element 110, will deform slightly as it touches the tip of the outlet 130 since the shifting element 110 moves to attach to the bladder outlet 130. Thus the tip of the outlet 130 sinks into the deformation element 120. At this time, the outlet 130 is covered by the deformation element 120, and thus air in the bladder may not be released through the outlet 130.

Referring to FIG. 1B, a schematic view of a conventional air pressure-adjusting device with the shifting element separated from the bladder outlet is shown. As described above, the outlet 130 is adjoined to the deformation element 120 so that air in the bladder may not be released from the outlet 130. If air in the bladder is to be released (i.e. the bladder is to be decompressed) so that the air pressure in the bladder may be lowered, the driving element should be used to drive the shifting element 110 to move away from the outlet 130 so that the deformation element 120 does not cover the outlet 130.

The shifting element 110, as driven by the driving element, may move back and forth along the shifting direction X1 as shown in FIG. 1A and FIG. 1B. The movement of the shifting element may force the deformation element 120 to cover the bladder outlet 130 and air in the bladder releases at a regular or desired rate. Therefore, air pressure in the bladder may be lowered steadily, or air in the bladder may be released completely.

As described above, in the conventional air pressure-adjusting device, the outlet 130 is either opened completely when not covered by the deformation element 120 or closed completely when covered by the deformation element 120. Therefore, in practice, the air pressure-adjusting device often has the following disadvantages. First, the rate of releasing air from the bladder is difficult to control accurately, because the outlet is either completely opened or closed. Secondly, air in the bladder may only be released in an erratic way. Therefore, the reduction of air pressure in the bladder may not be controlled effectively, and the air-releasing rate may not be regulated. The improvement of the air pressure-adjusting device is disclosed in US Patent No.6346082. There are provided a casing, a valve element that effects opening/closure of an evacuation port, and means for driving that effect opening/closure drive of this valve element. Fine irregularities are formed on valve element. The means for driving comprise: a cylindrical yoke; a core provided with a cylindrical part arranged within the yoke and a flange that is abutted by one end of the yoke; a magnet arranged within the yoke and that forms magnetic flux in the radial direction; a movable element arranged through the cylindrical part of the core with the valve element mounted at one end; a coil arranged in the gap between the magnet and yoke and fixed at one end of the movable element; and holding members that hold both ends of the movable element and that hold the movable element and coil in non-contacting condition with respect to the core and yoke. Other improvement of the air pressure-adjusting device still needs to be done.

### SUMMARY OF THE INVENTION

It is therefore an object of the invention to provide an air pressure-adjusting device in order to accurately control the rate of air pressure reduction in the bladder of a blood pressure monitor.

The invention achieves the above-identified object by providing an air pressure-adjusting device according to claim 1.

Other objects, features, and advantages of the invention will become apparent from the following detailed description of the preferred but non-limiting embodiments. The following description is made with reference to the accompanying drawings.

### BRIEF DESCRIPTION OF THE DRAWINGS

FIG. 1A (Prior Art) is a schematic view of a conventional air pressure adjusting device with the shifting element adjoining the bladder outlet;

FIG. 1B (Prior Art) is a schematic view of a conventional air pressure adjusting device with the shifting element separated from the bladder outlet;

FIG. 2A is a schematic view of the air pressure adjusting device according to a preferred embodiment of the invention;

FIG. 2B is a schematic view of the movement of the shifting element along a shifting direction to reduce the deflection of the deformation element in a decompressing process;

FIG. 2C is a schematic view of the deformation of the deformation element to cover the outlet again with the shifting element located at that position as in FIG. 2B.

### DETAILED DESCRIPTION OF THE INVENTION

The deformation of the deformation element, according to the air pressure-adjusting device of the present invention, provides a force to interact with the air pressure in a container. Both the force exerted by the deformation element onto the outlet and the air pressure controls the release amount of the air and the consequential air pressure. The air pressure-adjusting device of the invention may be applied to any apparatus requiring the control of a gas or liquid releasing process, such as a blood pressure monitor. However, of course, this invention is not limited to be applied to a blood pressure monitor.

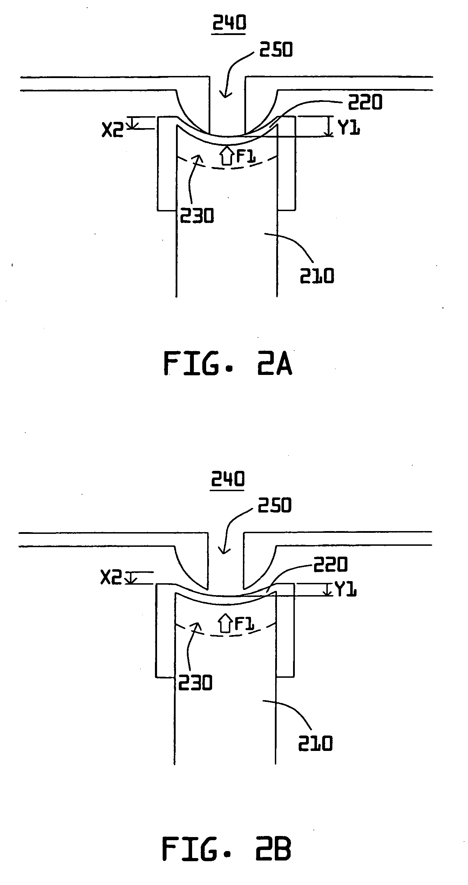

Referring to FIG. 2A, a schematic view of the air pressure-adjusting device according to a preferred embodiment of the invention is shown. The air pressure-adjusting device includes a driving element (not shown in FIG. 2A), a shifting element 210 having a cavity 230, and a deformation element 220. The air pressure-adjusting device is applicable to the adjustment of air pressure in a container 240 having an outlet 250 for releasing air.

The shifting element 210, coupled to the driving element, may move along a shifting direction X2 by a displacement when driven by the driving element. The deformation element 220 is disposed on the cavity 230 of the shifting element 210.

At first, the deformation element 220 will be driven by the shifting element 210 to cover the outlet 250 and deform at the cavity 230. As shown in FIG. 2A, the deformation element 220 will exert a force F1 on the outlet 250 corresponding to its deflection Y1. As the deformation element 220 abuts more and more closely to the outlet 250, the deflection Y1 of the deformation element 220 will be increase. Therefore, the force F1 exerted on the outlet 250, which is generated by the deflection Y1, will also increase until it is larger than the force exerted on the deformation element 220 by air pressure in the container 240. At this time, the deformation element 220 may cover and seal the outlet 250 completely to prevent air releasing out of the container 240.

Referring to FIG. 2B, a schematic view of the movement of the shifting element along a shifting direction to reduce the deflection of the deformation element in a decompressing process is shown. Subsequently, as the air pressure in the container 240 is lowered, the shifting element 210 is driven by the driving element to make a displacement along the shifting direction X2 (away from the outlet 250), which reduces the deflection Y1 of the deformation element 220. Therefore, the force F1 exerted on the outlet 250 generated by the deflection Y1 of the deformation element 220 will be decreased. The deformation element 220 does not disengage from the outlet 250 until the force F1 is less than the force exerted on the deformation element 220 by air in the container 240. At this time, air in the container 240 may be released by way of the outlet 250 (as shown in FIG 2B)

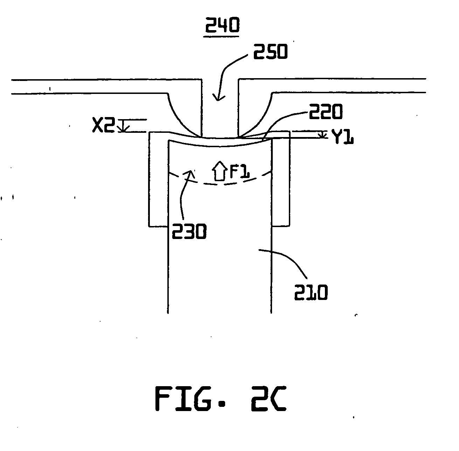

Referring to FIG. 2C, a schematic view of the deformation of the deformation element to again cover the outlet with the shifting element located at that position as in FIG. 2B is shown. In the decompressing process, air pressure in the container 240 is lowered gradually as air is released. The force exerted on the deformation element 220 decreases as the air pressure in the container 240 decreases and, at one point, it will be in equilibrium with the force F1 generated by the deflection Y1 of the deformation element 220. At this time, the deformation element 220 will again cover the outlet 250 and air in the container 240 will no longer be released.

As mentioned above, the driving element drives the shifting element 210 to move along the shifting direction X2 (away from the outlet 250) gradually. This increasing displacement reduces the deflection Y1 of the deformation element 220, thereby decreasing the force F1. On the other hand, decreasing air pressure in the container 240 reduces the force exerted on the deformation element 220 by air in the container 240. As a result of the changing balance between these two forces, the speed of releasing air out of the container 240 (i.e. the rate of reduction of air pressure in the container 240) may be regulated accurately. That is, in the decompressing process, the location of the shifting element 210 along the direction X2 may be quantified to represent the air pressure in the container 240 to some extent.

The container 240 in the invention may be a bladder of a blood pressure monitor, and the driving element may be any driving device, such as an electromagnetic coil, which may control and drive the movement of the shifting element.

As described above, the main feature of the air pressure-adjusting device in the invention is that the deformation element may be forced to make a deflection when compressed by the shifting element, which generates a corresponding force on the outlet. As a result of the changing balance between the force exerted by the deformation element and the force exerted on the deformation element by air in the container, air pressure in the container may be regulated very accurately. Therefore, the lowering rate of air pressure in the container may be also controlled very accurately. In the typical decompressing process, the outlet may be opened and closed repeatedly by driving the shifting element to move back and forth, so air in the bladder may be released and air pressure in the cuff may be regulated. In this invention, as the container is to be decompressed, the shifting element is driven to move along the shifting direction away from the outlet only, not to move back and forth as in the prior art. The air pressure is adjusted by the different degrees of deflection of the deformation element resulting from the one-way displacement of the shifting element. By the deflection of the deformation element, air in the bladder may be controlled to release more steadily, and the air pressure in the bladder may be regulated more precisely. In other words, in the decompressing process, the displacement of the shifting element may be quantified to represent the air pressure in the bladder to some extent.

The air pressure-adjusting device according to the preferred embodiments of the invention has the following advantage: the lowering rate of air pressure in the bladder may be controlled accurately. Air in the container may be controlled to release constantly; that is, the process for controlling the rate of releasing air may be according to the air pressure-adjusting device in the invention.

While the invention has been described by way of example and in terms of a preferred embodiment, it is to be understood that the invention is limited only by the scope of the appended claims.

## Claims

1. An air pressure-adjusting device for a container (240) having an outlet (250) for releasing air, the device comprising:
- a driving element;
- a shifting element (210), having a cavity (230), wherein the shifting element (210) is coupled to the driving element, and is driven by the driving element to make a displacement; and
- a deformation element (220), disposed at the cavity (230) of the shifting element (210);
- wherein the deformation element (220) is adapted to deform into the cavity (230) with an increasable/reducible deflection (Y1) so as to exert a force to cover partially or completely the outlet (250) according to the degree of displacement of the shifting element (210), thereby controlling the amount of air released from the container (240) to adjust the air pressure in the container (240) according to the force and the air pressure in the container (240).

2. The air pressure-adjusting device according to claim 1, wherein the driving element is an electromagnetic coil.

## Patentansprüche

1. Luftdruck-Einstellvorrichtung für einen Container (240) mit einem Auslass (250) um Luft abzulassen, wobei die Vorrichtung aufweist:
- ein Antriebselement;
- ein Verschiebeelement (210) mit einer Vertiefung (230), wobei das Verschiebeelement (210) mit dem Antriebselement verbunden ist und durch das Antriebselement angetrieben wird, um eine Verschiebung auszuführen; und
- ein Verformungselement (220), das an der Vertiefung (230) des Verschiebeelements (210) angeordnet ist;
- wobei das Verformungselement (220) mit einer erhöhbaren/reduzierbaren Durchbiegung (Y1) in die Vertiefung (230) verformt werden kann, um eine Kraft auszuüben, um den Auslass (250) entsprechend einem Grad der Verschiebung des Verschiebeelements (210) teilweise oder ganz abzudecken, um dadurch die Menge der aus dem Container (240) abgelassenen Luft zu regeln, um den Luftdruck im Container (240) entsprechend der Kraft und dem Luftdruck im Container (240) einzustellen.

2. Luftdruck-Einstellvorrichtung nach Anspruch 1, wobei das Antriebselement eine elektromagnetische Spule ist.

## Revendications

1. Dispositif de réglage de la pression de l'air destiné à un récipient (240) comportant un orifice de sortie (250) permettant de laisser échapper l'air, le dispositif comprenant :
- un élément d'entraînement ;
- un élément mobile (210) présentant une cavité (230), l'élément mobile (210) étant couplé à l'élément d'entraînement et étant entraîné par l'élément d'entraînement pour effectuer un déplacement ; et
- un élément à déformation (220), disposé au niveau de la cavité (230) de l'élément mobile (210) ;
- l'élément à déformation (220) étant adapté à se déformer dans la cavité (230) selon une flèche (Y1) susceptible d'être augmentée/réduite afin d'exercer une force pour couvrir partiellement ou complètement l'orifice de sortie (250) en fonction du degré de déplacement de l'élément mobile (210), commandant ainsi la quantité d'air échappé du récipient (240) afin de régler la pression de l'air à l'intérieur du récipient (240) en fonction de la force et de la pression de l'air dans le récipient (240).

2. Dispositif de réglage de la pression de l'air selon la revendication 1, dans lequel l'élément d'entraînement est une bobine électromagnétique.
